# EUROPEAN PATENT APPLICATION

(11) **EP 4 570 228 A1**
(43) Date of publication of application: **18.06.2025**
(21) Application number: 23852945.7
(22) Date of filing: 08.08.2023
(51) Int. Cl.: A61H 31/00, A61H 37/00

(54) **HEAD-UP CARDIOPULMONARY RESUSCITATION DEVICE BASED ON CEREBRAL BLOOD FLOW MEASUREMENT, AND METHOD FOR OPERATING SAME**

(30) Priority: 12.08.2022 KR 20220101391
(71) Applicant: Seoul National University Hospital, Seoul 03080 (KR)
(72) Inventor: LEE, Dong Keon, Seongnam-si, Gyeonggi-do 13597 (KR); KIM, Dae Kon, Seongnam-si, Gyeonggi-do 13620 (KR)
(74) Representative: Dentons Patent Solutions Rechtsanwaltsgesellschaft mbH
(86) International application number: PCT/KR2023/011672
(87) International publication number: WO 2024/035073

(57) **Abstract**

Provided are a cardiopulmonary resuscitation device, and a method for operating the cardiopulmonary resuscitation device. The cardiopulmonary resuscitation device may comprise: a cardiopulmonary resuscitation board comprising a head support part, which includes a receiving portion formed concavely in order to support at least a portion of a patient's head, a support, which is disposed at the bottom of the head support pat and adjusts the angle of the patient's head, a backboard for supporting the patient's back, and a belt for securing the patient; a cerebral blood flow measurement device for measuring the real-time cerebral blood flow of the patient according to the angle of the patient's head; and a control unit for controlling to adjust the angle between the support and the backboard within a preset angle range on the basis of the measured cerebral blood flow of the patient.

## Description

### Technical field

The present disclosure relates to a cardiopulmonary resuscitation device and a method for operating the same. Specifically, the present disclosure relates to a cardiopulmonary resuscitation device capable of allowing a patient to take a head-up posture and performing emergency treatment based on cerebral blood flow measurement of the patient in the head-up posture, and a method for operating the cardiopulmonary resuscitation device.

### Background Art

Acute cardiac arrest is one of the diseases that poses a large burden on public health due to its increasing incidence every year and low survival rate. According to the Korea Centers for Disease Control and Prevention (KCDC), the survival rate of psychogenic acute cardiac arrest in Korea increased from 2.3% in 2007 to 5.5% in 2017, and the brain function recovery rate thereof increased from 0.6% in 2007 to 3.1% in 2017. However, most patients with acute cardiac arrest still die or develop severe neurological disorders. In particular, only 3.0% of the patients survived prehospital cardiac arrest and only 0.9% of the patients neurologically recovered. Therefore, the posture of a patient during cardiopulmonary resuscitation in a cardiopulmonary resuscitation mode has become an important topic through several studies.

Chest compression in the supine position, which is a standard cardiopulmonary resuscitation chest compression position, is commonly performed for cardiopulmonary resuscitation position. As the chest compression in the supine position for cardiopulmonary resuscitation is performed, the blood volume and the pressure in the arterial system and the right heart are increased and transmitted to the brain through the jugular vein and the paravertebral venous plexus, thereby increasing the intracranial pressure and decreasing the brain perfusion. Accordingly, the intracranial pressure is typically high in patients in a supine position, and patients with traumatic brain injury are typically subjected to a head-raising posture in a non-invasive manner to reduce the intracranial pressure.

In other words, cardiopulmonary resuscitation in a head-up posture, which is the head-raising posture, increases the venous return from the brain to the heart by gravity, reduces the intracranial pressure, and improves the cardiac preload. In addition, several recent studies have shown that the head-up cardiopulmonary resuscitation reduces perfusion of the intracranial pressure, thereby increasing the cerebral perfusion pressure and the cerebral blood flow.

Accordingly, there is a need for a cardiopulmonary resuscitation device that enables a patient with cardiac arrest to take a stable head-up posture and performs precise emergency treatment by measuring the cerebral blood flow of the patient in real time.

### Detailed Description of the Invention

### Technical Problem

The technical problem to be solved by the present disclosure is to provide a cardiopulmonary resuscitation device that enables a patient to take a stable head-up posture and performs precise emergency treatment by measuring the cerebral blood flow of the patient in real time.

Another technical problem to be solved by the present disclosure is to provide a method for operating the cardiopulmonary resuscitation device that enables a patient to have a stable head-up posture and performs precise emergency treatment by measuring the cerebral blood flow of the patient in real time.

The technical problems of the present disclosure are not limited to the above-mentioned technical problems, and other technical problems that are not mentioned will be clearly understood by those skilled in the art from the following description.

### Technical Solution

A cardiopulmonary resuscitation device according to some embodiments for achieving the above technical problem may include: a cardiopulmonary resuscitation board including a head support part, which includes a receiving portion formed concavely in order to support at least a portion of a patient's head, a support, which is disposed at the bottom of the head support part and adjusts the angle of the patient's head, a backboard for supporting the patient's back, and a belt for securing the patient; a cerebral blood flow measurement device for measuring the real-time cerebral blood flow of the patient according to the angle of the patient's head; and a control unit for controlling to adjust the angle between the support and the backboard within a preset angle range on the basis of the measured cerebral blood flow of the patient.

The preset angle range may be 10 degrees to 45 degrees.

The control unit may operate in either a first mode or a second mode different from each other, and secure the support when the real-time cerebral blood flow of the patient satisfies a predetermined condition according to the mode of the control unit.

When the control unit operates in the first mode, the control unit may adjust the support so that the angle between the support and the backboard gradually increases, and secure the support at a specific angle when the real-time cerebral blood flow of the patient at the specific angle has a value greater than a predetermined target cerebral blood flow.

The predetermined target cerebral blood flow may be 1.15 times the real-time cerebral blood flow of the patient when the angle between the support and the backboard is 0 degree.

When the control unit operates in the second mode, the control unit may adjust the support so that the angle between the support and the backboard corresponds to each of first to N^{th} angles within the preset angle range, and secure the support at an angle at which the greatest increase in cerebral blood flow is exhibited among the first to N^{th} angles, wherein an i^{th} angle (i is a natural number between 2 and N) among the first to N^{th} angles may be greater than an i-1^{th} angle, the first angle may be a minimum value of the preset angle range, and the N^{th} angle may be a maximum value of the preset angle range.

The difference between the i^{th} angle and the i-1^{th} angle may have a constant value.

The cerebral blood flow measurement device may include an inspection patch attached to an occipital region of the patient to obtain a cerebral blood flow signal.

The cerebral blood flow measurement device may be a near-infrared spectroscopy (NIRS) device.

A method for operating a cardiopulmonary resuscitation device according to some embodiments for accomplishing the above technical problem may include: adjusting an angle between a back of a patient and a head of the patient using a support, according to an operation mode of a control unit; measuring a real-time cerebral blood flow of the patient according to an angle of the head of the patient by using an inspection patch attached to the occipital region of the patient to obtain a cerebral blood flow signal; searching for an angle satisfying a predetermined condition according to an operation mode of the control unit; and securing the support at an angle satisfying the predetermined condition, wherein the searching for the angle satisfying the predetermined condition may be based on determining whether the measured real-time cerebral blood flow of the patient at least at any angle has a value greater than the predetermined cerebral blood flow.

The angle between the patient's back and the patient's head may be adjusted between 10 degrees and 45 degrees.

The predetermined target cerebral blood flow may be 1.15 times a real-time cerebral blood flow of the patient when the angle between the patient's back and the patient's head is 0 degree.

The operation mode of the control unit may include: a first mode for adjusting the support so that the angle between the patient's back and the patient's head gradually increases; and a second mode for adjusting the support so that the angle between the patient's back and the patient's head corresponds to each of first to N^{th} angles within the preset angle range, wherein the i^{th} angle (i is a natural number between 2 and N) among the first to N^{th} angles may be greater than the i-1^{th} angle, the first angle may be a minimum value of the preset angle range, and the N^{th} angle may be a maximum value of the preset angle range.

When the control unit operates in the second mode, the searching for an angle satisfying the predetermined condition may include searching for an angle at which the greatest increase in cerebral blood flow is exhibited among the first to N^{th} angles.

The real-time cerebral blood flow of the patient may be measured by the NIRS device.

The details of other embodiments are included in the detailed description and the drawings.

### Advantageous Effects

According to some embodiments of the present disclosure, a cardiopulmonary resuscitation device and a method for operating the same may stably position a head of a patient higher than a heart of the patient to increase a brain perfusion pressure and a cerebral blood flow, thereby improving a neurological prognosis of the patient, and may measure a cerebral blood flow of the patient in real time according to an angle of the head of the patient, thereby determining a posture of the patient for effective cardiopulmonary resuscitation.

### Brief Description of the Drawings

FIG. 1 is an exemplary block diagram of a cardiopulmonary resuscitation device according to some embodiments.
FIG. 2 is an exemplary diagram of a cardiopulmonary resuscitation board according to some embodiments.
FIG. 3 is an exemplary diagram of a receiving portion according to some embodiments.
FIG. 4 is an exemplary diagram of a cerebral blood flow measurement device according to some embodiments.
FIG. 5 is an exemplary diagram illustrating attachment locations of inspection patches according to some embodiments.
FIG. 6 is a flowchart of a method for operating the cardiopulmonary resuscitation device according to some embodiments.
FIG. 7 is a flowchart illustrating an operation mode of a control unit according to some embodiments.
FIG. 8 is a flowchart illustrating an operation mode of the control unit according to other embodiments.

### Detailed Description of the Embodiments

Hereinafter, preferred embodiments of the present disclosure will be described in detail with reference to the accompanying drawings. Advantages and features of the present disclosure, and methods for achieving the same, will become apparent by referring to the following detailed description of embodiments taken in conjunction with the accompanying drawings. However, the present disclosure is not limited to the embodiments disclosed below. The present disclosure may be implemented in various forms. The present embodiments are only provided to make the present disclosure complete and to fully convey the scope of the present disclosure to those skilled in the art. The present disclosure is only defined by the scope of the claims. The same reference numerals refer to the same components throughout the specification.

The terminology used herein is only for describing the embodiments and is not intended to limit the present disclosure. As used herein, the singular forms include plural referents unless the context clearly dictates otherwise. As used herein, terms such as "comprises" and/or "comprising" do not exclude the presence or addition of one or more other components, steps, operations, and/or elements.

Unless otherwise defined, all terms (including technical and scientific terms) used herein have the same meaning as commonly understood by those skilled in the art to which the present disclosure belongs. In addition, terms defined in commonly used dictionaries are not to be interpreted ideally or unduly unless explicitly defined.

Hereinafter, embodiments of the present disclosure will be described in detail with reference to the accompanying drawings. In describing with reference to the drawings, the same or corresponding components will be denoted by the same reference numerals regardless of the drawing numbers, and repeated description thereof will be omitted.

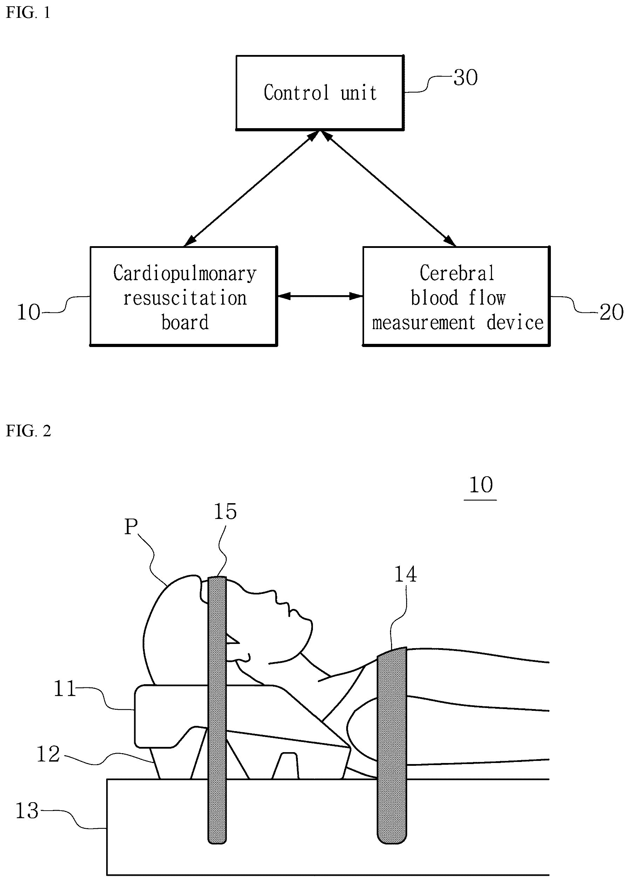

FIG. 1 is an exemplary block diagram of a cardiopulmonary resuscitation device according to some embodiments.

Referring to FIG. 1, a cardiopulmonary resuscitation device 1 may include a cardiopulmonary resuscitation board 10, a cerebral blood flow measurement device 20, and a control unit 30.

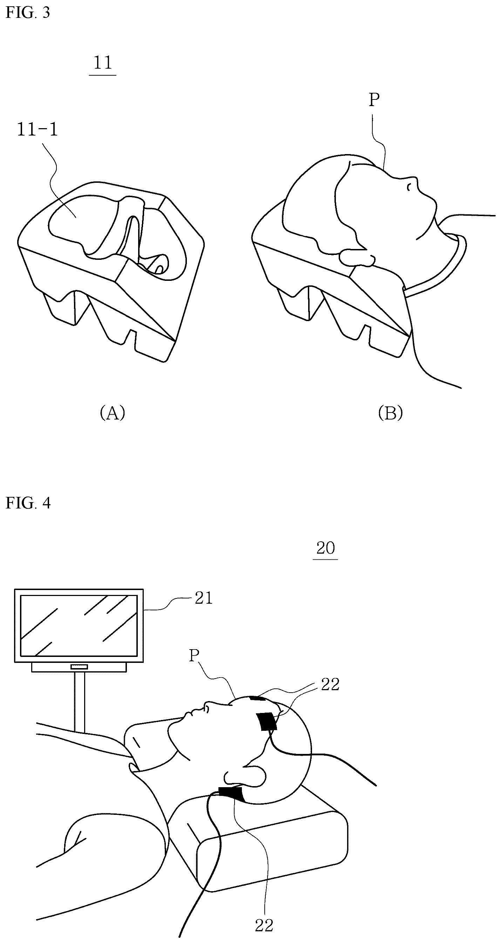

The cardiopulmonary resuscitation board 10 may allow a patient to assume a stable head-up posture during cardiopulmonary resuscitation through the various configurations described with reference to FIG. 2. Details thereof are described with reference to FIG. 2.

The cerebral blood flow measurement device 20 may measure the patient's real-time cerebral blood flow. Specifically, the cerebral blood flow measurement device 20 may measure the real-time cerebral blood flow of the patient according to an angle of the head of the patient.

The angle of the patient's head may refer to an angle between the patient's back and the patient's neck. Specifically, the angle of the patient's head may refer to an angle between the patient's back and the start of the neck toward the patient's shoulder when the patient's back is in close contact with the backboard of the cardiopulmonary resuscitation board 10.

The cerebral blood flow measurement device 20 may provide information on the measured real-time cerebral blood flow of the patient to the control unit 30.

The control unit 30 may control the operation of the cardiopulmonary resuscitation board 10 based on the measured cerebral blood flow of the patient. Specifically, the control unit 30 may control the cardiopulmonary resuscitation board 10 to adjust the angle of the patient's head within a preset angle range. In this case, the preset angle range may be 10 degrees to 45 degrees, but the embodiment is not limited thereto. The preset angle range may be variously changed according to the embodiment.

More specifically, the control unit 30 may receive information on the measured real-time cerebral blood flow of the patient from the cerebral blood flow measurement device 20, and control the cardiopulmonary resuscitation board 10 to fix the angle of the patient's head to a specific angle when the real-time cerebral flow satisfies a predetermined condition according to an operation mode.

For example, the control unit 30 may operate in either a first mode or a second mode. When the control unit 30 operates in the first mode, the control unit 30 may control the cardiopulmonary resuscitation board 10 so that the angle of the patient's head gradually increases. While the control unit 30 operates in the first mode so that the angle of the head of the patient gradually increases, if the real-time cerebral blood flow of the patient at a specific angle has a value greater than the predetermined target cerebral blood flow, the control unit 30 may control the cardiopulmonary resuscitation board 10 to be fixed at the corresponding specific angle.

On the other hand, the target cerebral blood flow may be 1.15 times greater than the real-time cerebral blood flow of the patient, for example, when the angle between the patient's back and the patient's head is 0 degree. However, the embodiments are not limited thereto. The target cerebral blood flow may be variously changed according to the embodiments.

On the other hand, when the control unit 30 operates in the second mode, the control unit 30 may control the cardiopulmonary resuscitation board 10 so that the angle of the head of the patient corresponds to each of the first to N^{th} angles within the above-described preset angle range. While the control unit 30 operates in the second mode so that the angle of the patient's head corresponds to each of the first through N^{th} angles, the cerebral blood flow of the patient at each angle may be measured by the cerebral blood flow measurement device 20 at each angle. At this time, the control unit 30 may control the cardiopulmonary resuscitation board 10 to be fixed at the angle at which the greatest increase in cerebral blood flow is exhibited among the first to N^{th} angles.

On the other hand, an i^{th} angle (i is a natural number between 2 and N) among the first to N^{th} angles may be greater than the i-1^{th} angle, the first angle may be a minimum value of the preset angle range, and the N^{th} angle may be a maximum value of the preset angular range. That is, when the preset angle range is 10 degrees to 45 degrees as described above, the first angle may refer to 10 degrees, and the N^{th} angle may refer to 45 degrees.

In addition, a difference between the i^{th} angle and the i-1^{th} angle may have a constant value. For example, when the preset angle range has the above-described value and the difference between the i^{th} angle and the i-1^{th} angle is 5 degrees, the control unit 30 may control the cardiopulmonary resuscitation board 10 so that the angle of the head of the patient corresponds to each of 10 degrees, 15 degrees, 20 degrees, 25 degrees, 30 degrees, 35 degrees, 40 degrees, and 45 degrees.

However, the embodiment is not limited thereto. The first to N^{th} angles may be variously changed according to the present angle range and the difference between the i^{th} angle and the i-1^{th} angle. For example, when the preset angle range is 15 degrees to 35 degrees and the difference between the i^{th} angle and the i-1^{th} angle is 10 degrees, the control unit 30 may control the cardiopulmonary resuscitation board 10 so that the angle of the head of the patient corresponds to each of 15 degrees, 25 degrees, and 35 degrees.

Although FIG. 1 shows that the control unit 30 is separate from the cardiopulmonary resuscitation board 10 and the cerebral blood flow measurement device 20, the embodiment is not limited thereto. For example, the control unit 30 may be integrally formed by being mounted inside the cardiopulmonary resuscitation board 10 or may be integrally formed by being mounted inside the cerebral blood flow measurement device 20.

FIG. 2 is an exemplary diagram of a cardiopulmonary resuscitation board according to some embodiments.

Referring to FIG. 2, the cardiopulmonary resuscitation board 10 may include a head support part 11, a support 12, a backboard 13, a shoulder securing belt 14, and a forehead securing belt 15.

The head support part 11 may be configured to support at least a part of the head of a patient P. The head support part 11 may be made of a fluffy material to minimize damage to the scalp even if the patient lies down for a long time of 30 minutes or more. The head support part 11 may include a receiving portion that is manufactured to be suitable for the head of a human body so that the occipital region can be suitably received in the head support part 11. Details thereof are described with reference to FIG. 3.

The support 12 may be disposed below the head support part 11 to adjust the angle of the head of the patient P.

The backboard 13 may support the back of the patient P. The backboard 13 may be configured to provide a firm repulsive force to the lower back of the patient during cardiopulmonary resuscitation.

The shoulder securing belt 14 may secure the shoulder of the patient P. The forehead securing belt 15 may secure the head of the patient P. By using the shoulder securing belt 14 and the forehead securing belt 15 to firmly secure the head and other body parts of the patient P that may be shaken due to emergency treatment, the patient P may receive stable cardiopulmonary resuscitation.

Meanwhile, the support 12 may adjust the angle of the head of the patient P under the control of the control unit 30 of FIG. 1. Specifically, to increase the angle of the head of the patient P, the support 12 may increase the inclination of its surface in contact with the head support part 11 under the control of the control unit 30. To decrease the angle of the head of the patient P, the support 12 may decrease the inclination of its surface in contact with the head support part 11 under the control of the control unit 30.

Meanwhile, the angle of the head of the patient P may refer to an angle between the backboard 13 and the surface of the support 12 in contact with the head support part 11. That is, the angle between the backboard 13 and the surface of the support 12 in contact with the head support part 11 may correspond to the angle between the back of the patient P and the neck of the patient P described with reference to FIG. 1.

FIG. 3 is an exemplary diagram of a receiving portion according to some embodiments.

Referring to FIG. 3, the head support part 11 may include a receiving portion 11_1. Specifically, as illustrated in FIG. 3 (A), the head support part 11 may include the receiving portion 11_1 formed to be concave to support at least a part of the head of the patient P. Accordingly, as illustrated in FIG. 3 (B), it is possible to provide an environment in which the patient P may take a stable posture through the receiving portion 11_1. On the other hand, the shape of the receiving portion 11_1 is not necessarily limited to the shape shown in FIG. 3. Depending on the embodiment, the shape of the receiving portion 11_1 may be changed to various shapes capable of stably and appropriately supporting at least a part of the patient's head.

FIG. 4 is an exemplary diagram of a cerebral blood flow measurement device according to some embodiments.

Referring to FIG. 4, the cerebral blood flow measurement device 20 may include a cerebral blood flow analysis device 21 and an inspection patch 22.

The cerebral blood flow analysis device 21 may analyze a cerebral blood flow signal received from the inspection patch 22 to analyze the real-time cerebral blood flow of the patient P. In this case, the cerebral blood flow analysis device 21 may analyze the cerebral blood flow using near-infrared spectroscopy (NIRS). However, the embodiment is not limited thereto. The cerebral blood flow analysis device 21 may measure the cerebral blood flow of the patient P through any other appropriate methods according to the embodiment. In addition, the cerebral blood flow analysis device 21 may use a micro light emitted diode (LED) to obtain an improved cerebral blood flow signal of the patient P, but the embodiment is not necessarily limited thereto.

The inspection patch 22 may be attached to the skin of the patient P to obtain the cerebral blood flow signal. For example, the inspection patch 22 may be attached to the forehead of the patient P, or may be attached to the occipital region of the patient P.

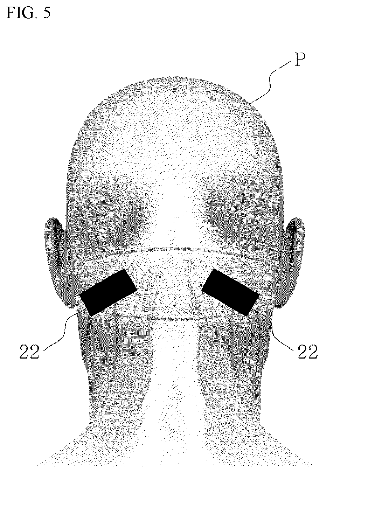

FIG. 5 is an illustrative diagram for describing attachment locations of inspection patches according to some embodiments.

Referring to FIG. 5, the inspection patch 22 may be attached not only to the forehead but also to the occipital region of the patient P. In the case of a general inspection patch attached to both sides of the forehead of the patient P to obtain a signal, there may be some problems in obtaining a signal from a patient receiving cardiopulmonary resuscitation, a patient with decreased consciousness, or a patient who is conscious, but has sweat or other causes that result in frequent poor adhesion.

However, the inspection patch 22 according to some embodiments of the present disclosure may be attached to the occipital region of the patient P, which exhibits better adhesion than the forehead due to the weight of the head of the patient P. Accordingly, the cerebral blood flow signal of the patient P may be more effectively obtained.

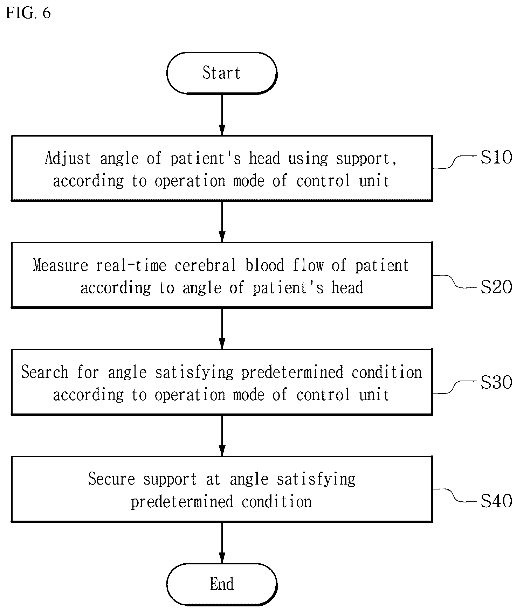

FIG. 6 is a flowchart of a method for operating the cardiopulmonary resuscitation device according to some embodiments.

Referring to FIG. 6, the cardiopulmonary resuscitation device may adjust the angle of the head of the patient by using the support according to the operation mode of the control unit (S10). Specifically, as described with reference to FIGS. 1 to 5, the control unit may adjust the angle of the support of the cardiopulmonary resuscitation board, thereby adjusting the angle of the head of the patient.

Then, the real-time cerebral blood flow of the patient may be measured according to the angle of the head of the patient (S20). Specifically, when the head of the patient is positioned to have an angle according to the operation mode of the control unit, the real-time cerebral blood flow of the patient may be measured.

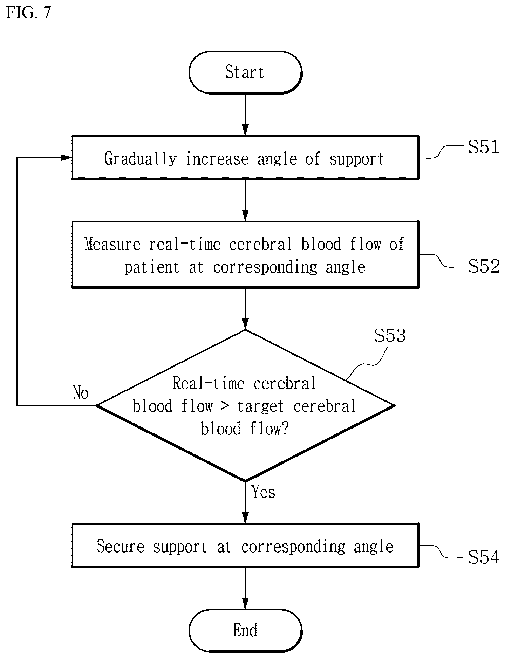

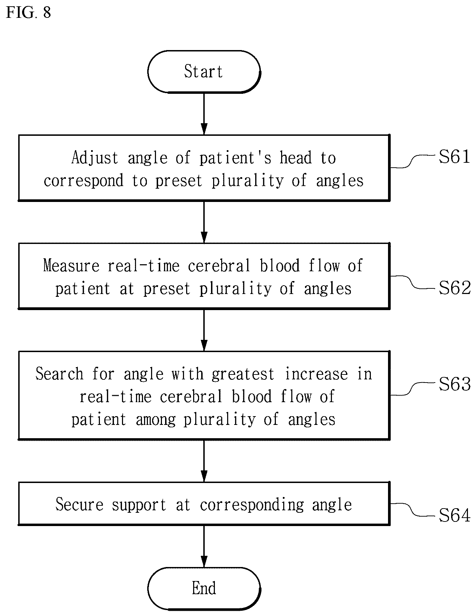

Then, an angle satisfying a predetermined condition according to the operation mode of the control unit may be searched for (S30). Specifically, it is possible to search for an angle satisfying a predetermined condition according to the first mode of the control unit or a predetermined condition according the second mode. Details thereof are described below with reference to FIG. 7 and FIG. 8.

Then, the support may be fixed at an angle satisfying the predetermined condition (S40).

FIG. 7 is a flowchart illustrating an operation mode of a control unit according to some embodiments. Specifically, FIG. 7 is a flowchart illustrating an operation of the control unit according to the first mode.

Referring to FIG. 7, in the first mode, the control unit may gradually increase the angle of the support (S51). For example, the control unit may gradually increase the angle of the support from the minimum value of the preset angle range. However, the embodiment is not limited thereto. The control unit may gradually increase the angle of the support from a different value.

Then, the control unit may measure the real-time cerebral blood flow of the patient at gradually increasing angles (S52).

Then, the control unit may determine whether the real-time cerebral blood flow of the patient has a value greater than the target cerebral blood flow (S53).

If the real-time cerebral blood flow of the patient has a value smaller than the target cerebral blood flow (NO in S53), the control unit may further increase the angle of the support. On the other hand, when the real-time cerebral blood flow of the patient has a value greater than the target cerebral blood flow (Yes in S53), the control unit may control the support to be fixed at the corresponding angle (S54).

FIG. 8 is a flowchart illustrating an operation mode of the control unit according to other embodiments. Specifically, FIG. 8 is a flowchart illustrating an operation of the control unit according to the second mode.

Referring to FIG. 8, in the second mode, the control unit may adjust the angle of the head of the patient, that is, the angle of the support to correspond to a plurality of preset angles (S61). Since the plurality of preset angles are the same as those described with reference to FIG. 1, detailed description thereof will be omitted.

Then, the control unit may measure the real-time cerebral blood flow of the patient at the plurality of preset angles (S62).

Then, the control unit may search for an angle at which the greatest increase in cerebral blood flow is exhibited among the real-time cerebral blood flows of the patient at the plurality of angles (S63).

Then, the control unit may control the support to be fixed at an angle at which the greatest increase in cerebral blood flow is exhibited (S64).

Although the embodiments of the present disclosure have been described above with reference to the accompanying drawings, it should be understood that the present disclosure is not limited to the above embodiments but is manufactured in various different forms. Those skilled in the art may implement the present disclosure in other specific forms without modifying the technical concept or essential features of the present disclosure. It is therefore to be understood that the embodiments described above are illustrative and not restrictive in any respect.

### [Description of Signs]

1: Cardiopulmonary resuscitation device.
10: Cardiopulmonary resuscitation board.
11: Head support part
11_1: Receiving portion
12: Support
13: Backboard
14: Shoulder securing belt
15: Forehead securing belt
20: Cerebral blood flow measurement device
21: Cerebral blood flow analysis device
22: Inspection patch
P: Patient

## Claims

1. A cardiopulmonary resuscitation device comprising:
a cardiopulmonary resuscitation board comprising a head support part, which comprises a receiving portion formed concavely in order to support at least a portion of a patient's head, a support, which is disposed at the bottom of the head support part and adjusts the angle of the patient's head, a backboard for supporting the patient's back, and a belt for securing the patient;
a cerebral blood flow measurement device for measuring a real-time cerebral blood flow of the patient according to the angle of the patient's head; and
a control unit for controlling to adjust the angle between the support and the backboard within a preset angle range on the basis of the measured cerebral blood flow of the patient.

2. The cardiopulmonary resuscitation device of claim 1, wherein the preset angle range is 10 degrees to 45 degrees.

3. The cardiopulmonary resuscitation device of claim 1, wherein the control unit operates in either a first mode or a second mode different from each other, and secures the support when the real-time cerebral blood flow of the patient satisfies a predetermined condition according to the mode of the control unit.

4. The cardiopulmonary resuscitation device of claim 3, wherein, when the control unit operates in the first mode, the control unit adjusts the support so that the angle between the support and the backboard gradually increases, and secures the support at a specific angle when the real-time cerebral blood flow of the patient at the specific angle has a value greater than a predetermined target cerebral blood flow.

5. The cardiopulmonary resuscitation device of claim 4, wherein the predetermined target cerebral blood flow is 1.15 times the real-time cerebral blood flow of the patient when the angle between the support and the backboard is 0 degree.

6. The cardiopulmonary resuscitation device of claim 3, wherein, when the control unit operates in the second mode, the control unit adjusts the support so that the angle between the support and the backboard corresponds to each of first to N^{th} angles within the preset angle range, and secures the support at an angle at which the greatest increase in cerebral blood flow is exhibited among the first to N^{th} angles, wherein an i^{th} angle (i is a natural number between 2 and N) among the first to N^{th} angles is greater than an i-1^{th} angle, the first angle is a minimum value of the preset angle range, and the N^{th} angle is a maximum value of the preset angle range.

7. The cardiopulmonary resuscitation device of claim 6, wherein a difference between the i^{th} angle and the i-1^{th} angle has a constant value.

8. The cardiopulmonary resuscitation device of claim 1, wherein the cerebral blood flow measurement device comprises an inspection patch attached to an occipital region of the patient to obtain a cerebral blood flow signal.

9. The cardiopulmonary resuscitation device claim 1, wherein the cerebral blood flow measurement device is a near-infrared spectroscopy (NIRS) device.

10. A method for operating a cardiopulmonary resuscitation device, comprising:
adjusting an angle between a back of a patient and a head of the patient using a support, according to an operation mode of a control unit;
measuring a real-time cerebral blood flow of the patient according to an angle of the head of the patient by using an inspection patch attached to an occipital region of the patient to obtain a cerebral blood flow signal;
searching for an angle satisfying a predetermined condition according to an operation mode of the control unit; and
securing the support at an angle satisfying the predetermined condition,
wherein the searching for an angle satisfying the predetermined condition is based on determining whether the measured real-time cerebral blood flow of the patient at least at any angle has a value greater than the predetermined cerebral blood flow.

11. The method of claim 10, wherein the angle between the back and the head of the patient is adjusted between 10 degrees and 45 degrees.

12. The method of claim 10, wherein the predetermined target cerebral blood flow is 1.15 times the real-time cerebral blood flow of the patient when the angle between the patient's back and the patient's head is 0 degree.

13. The method of claim 10, wherein the operation mode of the control unit comprises:
a first mode for adjusting the support so that the angle between the back and the head of the patient gradually increases; and
a second mode for adjusting the support so that the angle between the back and
the head of the patient corresponds to each of first to N^{th} angles within the preset angle range,
wherein an i^{th} angle (i is a natural number between 2 and N) among the first to Nth angles is greater than an i-1^{th} angle, the first angle is a minimum value of the preset angle range, and the N^{th} angle is a maximum value of the preset angle range.

14. The method of claim 13, wherein, when the control unit operates in the second mode, the searching for an angle satisfying the predetermined condition comprises searching for an angle at which the greatest increase in cerebral blood flow is exhibited among the first to N^{th} angles.

15. The method of claim 10, wherein the real-time cerebral blood flow of the patient is measured by a near-infrared spectroscopy (NIRS) device.
